# NEUE EUROPÄISCHE PATENTSCHRIFT

(11) **EP 0 630 967 B2**
(45) Veröffentlichungstag und Bekanntmachung des Hinweises auf die Entscheidung über den Einspruch: **07.09.2005**
(45) Hinweis auf die Patenterteilung: 08.08.2001
(21) Anmeldenummer: 94109708.1
(22) Anmeldetag: 23.06.1994
(51) Int. Cl.: C12N 9/10, C12P 19/18, C12N 15/54

(54) **Cyclodextringlycosyltransferasen zur Produktion von gamma-Cyclodextrin**
Cyclodextrin glycosyltransferase for the production of gamma-cyclodextrin
Cyclodextrine transférase pour la production de gamma-cyclodextrine

(30) Priorität: 24.06.1993 DE 4321047; 22.07.1993 DE 4324650
(43) Veröffentlichungstag der Anmeldung: 28.12.1994
(73) Patentinhaber: Consortium für elektrochemische Industrie GmbH, 81379 München (DE)
(72) Erfinder: Schulz, Georg, Prof. Dr., D-79211 Denzlingen (DE); Candussio, Anton, Dr., D-81825 München (DE)

(56) Entgegenhaltungen:
- EP-A- 0 291 067
- WO-A-91/14770
- DATABASE WPI Week 8813, Derwent Publications Ltd., London, GB; AN 88-088335 & JP-A-63 039 597 (HAYASHIBARA BIOCHEM) 20. Februar 1988
- APPLIED AND ENVIRONMENTAL MICROBIOLOGY Bd. 58, Nr. 12 , Dezember 1992 , AM.SOC.MICROBIOL.,WASHINGTON,DC,US; Seiten 4016 - 4025 S. FUJIWARA ET AL. 'Cyclization characteristics of cyclodextrin glucanotransferase are conferred by the NH2-terminal region of the enzyme'
- AGRIC. BIOL. CHEM. Bd. 54, Nr. 1 , Januar 1990 , TOKYO, JAPAN; Seiten 197 - 201 T. KANEKO ET AL. 'Comparison of CD composition produced by chimeric CGTases'
- BIOTECHN. APPL. BIOCHEM. Bd. 12, Nr. 4 , August 1990 , ACADEMIC PRESS, NEW YORK, US; Seiten 387 - 396 J. HELLMAN ET AL. 'Effects of modifications at the C-terminus of cyclomaltodextrin glucanotransferase from Bacillus circulans var. alkalophilus on catalytic activity'
- DATABASE WPI Week 9313, Derwent Publications Ltd., London, GB; AN 93-103608 & JP-A-5 041 985 (OJI CORN STARCH CO LTD) 23. Februar 1993
- DATABASE WPI Week 9151, Derwent Publications Ltd., London, GB; AN 91-373420 & JP-A-3 251 183 (OJI CORN STARCH KK) 8. November 1991
- BIOCHEMISTRY Bd. 31, Nr. 37 , 22. September 1992 , AM. CHEM. SOC.,WASHINGTON,DC,US; Seiten 8740 - 8746 C. KLEIN ET AL. 'Catalytic center of cyclodextrin glycosyltransferase derived from X-ray structure analysis combined with site directed mutagenesis'
- J. FERMENTATION AND BIOENGENEERING Bd. 74, Nr. 6 , 1992 , SUITA, JAPAN; Seiten 345 - 351 N. KITAMOTO ET AL. 'Cloning and sequencing of the gene encoding cyclodextrin glucanotransferase from Bacillus sp. KC201'
- DATABASE WPI Week 9339, Derwent Publications Ltd., London, GB; AN 93-308317 & JP-A-5 219 948 (UOZUMI T) 31. August 1993
- JOURNAL OF BIOTECHNOLOGY Bd. 32, Nr. 3 , 1994 , ELSEVIER, AMSTERDAM, THE NETHERLANDS; Seiten 283 - 288 K.-A. SIN ET AL. 'Replacement of an amino acid residue of cyclodextrin glucanotransferase of Bacillus ohbensis doubles the production of gamma-cyclodextrin'
- DATABASE WPI Week 9344, Derwent Publications Ltd., London, GB; AN 93-347473 & JP-A-5 244 945 (NIPPON SHOKUHIN KAKO KK) 24. September 1993

## Beschreibung

Die Erfindung betrifft Cyclodextringlycosyltransferasen (CGTasen) EC 2.4.1.19 zur Produktion von γ-Cyclodextrin, Verfahren zu ihrer Herstellung und ihre Verwendung.

Cyclodextrine werden in der Regel aus Stärke oder stärkeähnlichen Substraten hergestellt. Stärke wird dabei mit CGTasen enzymatisch in Cyclodextrin (CD) umgewandelt. Aus thermodynamischen Gründen wird die Stärke unabhängig von der zur Umsetzung verwendeten CGTase hauptsächlich in β-CD umgewandelt, wenn die Reaktion bis zum Erreichen des thermodynamischen Gleichgewichts (maximaler CD-Ertrag) durchgeführt wird. In der Initialphase, zu Beginn der Stärkekonversionsreaktion jedoch unterscheiden sich die zur Konversion verwendeten Enzyme in der Zusammensetzung des primären Produktgemisches. In Abhängigkeit von dem in dieser Initialphase durch das Enzym hauptsächlich gebildeten Produkt, α-, β-oder γ-CD, wird unterschieden zwischen α-, β-oder γ-CGTasen.

Solche zur industriellen CD-Produktion geeigneten und auch bereits verwendeten Enzyme wurden bisher ausschließlich bei Bakterien nachgewiesen. α-CGTasen wurden bisher ausschließlich bei Bacillus macerans (J. Bacteriol. (1986) 166, S. 1118-1122), Bacillus Stearothermophilus (GB 2169902) und Klebsiella oxytoca (Gene (1986) 47, S. 269-277) identifiziert. β-CGTasen wurden zum Beispiel bei Bacillus circulans (Appl. Microbiol. Biotechnol. (1990) 34, S. 229-230), Bacillus megaterium (US-A 3,812,011), Bacillus ohbensis (JP 74124285), Micrococcus sp. (EP 0017242) und taxonomisch nicht exakt klassifizierten alkalophilen Bacillen wie Bacillus sp. 38-2 (J. Gen. Microbiol. (1988) 134, S. 97-105), 17-1 (Proceedings of the 4th International Symposium on Cyclodextrins (1988), S. 87-92), 1011 (Appl. Microbiol. Biotechnol. (1987) 26, S. 149-153), 1-1 (Proceedings of the 4th International Symposium on Cyclodextrins (1988), S. 71-76) nachgewiesen. Enzyme mit einer initial hohen γ-CD-Bildungsaktivität wurden aus Bacillus subtilis 313 (Agric. Biol. Chem (1986) 50, S. 2161-2162), Bacillus sp. Al-6 (J. Ferment. Bioeng. (1990) 70 (3), S. 150-154) und Bacillus sp. 290-3 (Proceedings of the 4th International Symposium on Cyclodextrins (1988), S. 87-92) beschrieben.

Durch eine Röntgenstrukturanalyse wurde die dreidimensionale Struktur der β-CGTase aus Bacillus circulans, aufgeklärt (J. Mol. Biol. (1991) 217, S. 737-750). Aus dieser Struktur konnte abgeleitet werden, welche Aminosäurereste unmittelbar am Aufbau der Substratbindestelle und des aktiven Zentrums dieser CGTase beteiligt sein könnten, nicht jedoch, welche Aminosäurereste die Produktspezifität dieser CGTase determinieren (Biochemistry (1992) 31, S. 8740-8746).

Da die bei der industriellen Herstellung von Cyclodextrinen verwendeten CGTasen bei der Umwandlung von Stärke in Cyclodextrine immer Gemische aus mehreren Cyclodextrinen liefern, wurden verschiedene Verfahren zur Gewinnung reiner Cyclodextrine (α, β oder y) entwickelt:
- Definierte CD's können aus den Produktgemischen, z. B. aufgrund ihrer Molekulargewichtsunterschiede, chromatographisch abgetrennt werden (beschrieben beispielsweise in US-A 4808232).
- Bei der enzymatischen Umwandlung von Stärke in Cyclodextrine werden Komplexbildner zugesetzt, die nur mit einem definierten CD reagieren und damit z. B. einen unlöslichen Komplex bilden, der physikalisch aus dem Reaktionsgemisch abgetrennt werden kann. Anschließend wird der Komplex aufgelöst und das homogene CD gewonnen (beschrieben beispielsweise in EP 0291067).
- Durch die Zugabe eines organischen Lösungsmittels, wie z. B. Äthanol, zum Reaktionsgemisch kann die Produktzusammensetzung bei Verwendung einer γ-CGTase in Richtung γ-CD verschoben werden (J. Ferm. Bioeng. (1990) 70 (3), S. 150-154).

Bei jedem der Verfahren werden optimalerweise solche CGTasen verwendet, die eine möglichst hohe initiale Produktbildungspräferenz für das CD, besitzen, das rein hergestellt werden soll.

Die Spezifität der bisher bekannten α- und β-CGTasen ist ausreichend für eine technische Produktion der entsprechenden Cyclodextrine. Im Gegensatz dazu besitzt keine der bekannten γ-CGTasen eine Produktspezifität, die eine vergleichbare γ-CD-Produktion ermöglicht.

Zur Herstellung von γ-CD wurde daher in JP 03053892 vorgeschlagen, α- und/oder β-Cyclodextrine enzymatisch durch die Zugabe des γ-CD-spezifischen Komplexbildners Glycosylglycyrrhizin, Maltose und einer CGTase in γ-CD umzuwandeln.

In D1: DATABASE WPI Week 9313, Derwent Publications Ltd., London, GB; AN 93-103608 & JP -A-5 041 985 (OJI CORN STARCH CO LTD) 23. Februar 1993 wird eine CGTase ofenbart, die durch Mutagenisierung von Asp zu His In mehreren Aminosäurepositionen ihrer Sequenz vorzugsweise γ-CD produziert.

Aus Takeshi Uorumi et al, Sixth European Congress on Biotechnology, ECB6, Firenze, June 13-17, 1993 sind Mutationen eine CGTase aus B. ohbensis in Position 188 bekannt, wobei die Mutation von Tyr zu Trp zu einer Ausbeute von 15% γ-CD und 23% β-CD aus Stärke führt.

Aufgabe der Erfindung war es, weitere Cyclodextringlycosyltransferasen (CGTasen) zur Verfügung zu stellen, welche bei der Umsetzung von Stärke oder stärkeähnlichen Substraten zu CD in erhöhtem Ausmaß γ-CD produzieren.

Eine weitere Aufgabe der Erfindung war es, ein Verfahren zur Herstellung von CGTasen, welche bei der Umsetzung von Stärke oder stärkeähnlichen Substraten zu CD in erhöhtem Ausmaß γ-CD produzieren, zur Verfügung zu stellen.

Eine weitere Aufgabe der Erfindung war es, Verfahren zur Produktion von γ-CD mittels der genannten CGTasen zur Verfügung zu stellen.

Die Aufgabe der Erfindung wird gelöst durch mutierte CGTasen, gemäß Anspruch 1.

Die erfindungsgemäßen CGTasen produzieren bei der Umsetzung von Stärke oder stärkeähnlichen Substraten CD's in einer Produktverteilung bei der der Quotient aus γ-CD und der Summe aus α-CD und β-CD größer ist als der Quotient dieser Produkte, der bei der Stärkeumsetzung mit dem jeweiligen unveränderten Ausgangsenzym erzielt wird.

Unter Ausgangsenzym ist dabei die CGTase, die zur Herstellung der erfindungsgemäßen CGTase verwendet wurde, zu verstehen. Die erfindungsgemäßen CGTasen besitzen somit unerwarteterweise eine höhere γ-CD Spezifität als die zu ihrer Herstellung verwendeten Ausgangsenzyme.

Die erfindungsgemäßen CGTasen sind die aus den In Tab. 1 aufgeführten CGTasen durch Austausch des jeweils unterstrichenen Tyr gegen Trp oder Ser erhalten werden. Besonders bevorzugt sind CGTasen, bei denen das Tyr gegen Trp ausgetauscht ist.

**Tab. 1**

| CGTase aus Position Aminosäuresequenz | | |
|---|---|---|
| Typ Stamm | | |
| β B.circulans (SEQ. ID NO: 4) | 225 | Tyr Lys Asn Leu Tyr Asp Leu Ala Asp |
| γ B.sp. 290-3 (SEQ. ID NO: 8) | 207 | Tyr Arg Asn Leu Tyr Asp Leu Ala Ser |

Als Position ist in Tab. 1 die Zahl der ersten Aminosäure der jeweils wiedergegebenen Aminosäuresequenz bezeichnet, wobei als Position 1 die erste Aminosäure des Signalpeptids der jeweiligen CGTase Sequenz gezählt wurde.

Durch erfindungsgemäße Mutagenese des Tyr in diesen CGTasen mittels ebenfalls bekannter Standardverfahren, wie sie beispielhaft auch in der vorliegenden Anmeldung ausgeführt sind, lassen sich erfindungsgemäße Enzyme herstellen.

Eine weitere Aufgabe der Erfindung wird durch ein Verfahren gemäß Anspruch 3 gelöst. Das für eine CGTase kodierende Gen wird mittels bekannter Verfahren isoliert und die erfindungsgemäße Mutation wird in das Gen der CGTase durch "in vivo"- oder "in vitro" Mutageneseverfahren eingeführt.

Unter "in vivo"-Mutageneseverfahren sind besonders solche Methoden zu verstehen, bei denen Mikroorganismen, die chromosomal und/oder episomal ein für eine CGTase kodierendes Gen enthalten, mit einem Mutagen wie z. B. UV-Licht, Nitrosoguanidin oder Ethylmethylsulfonat unspezifisch mutagenisiert werden. Ein solches Verfahren ist beispielsweise von Miller J. H. in (172) Experiments in Molecular Genetics; Cold Spring Harbour Laboratory; Cold Spring Harbour, N.Y. beschrieben worden.

Anschließend werden durch bekannte Methoden wie beispielsweise der Sequenzanalyse nach der von Sanger et al. in PNAS 74 (1977) 5463-5467 beschriebenen Kettenabbruchmethode Mutanten identifiziert, bei denen zumindest das Codon des CGTase-Gens, welches für das Tyrosin, welches homolog zu Tyr 229 der CGTase aus Bacillus circulans ist, kodiert, durch ein Codon, welches für einen anderen natürlichen Aminosäurerest, bevorzugt einen Serin oder Tryptophanrest, besonders bevorzugt einen Tryptophanrest, kodiert, ersetzt ist.

Im Sinne der Erfindung sind unter Codons welche homolog zu Tyr 229 der CGTase aus Bacillus circulans sind die für Tyr kodierenden Codons anderer CGTase Gene zu verstehen die für das in Tab.1 unterstrichen Tyr in dem dort dargestellten Aminosäuresequenzmotiv kodieren.

Unter "in vitro"-Mutagenesemethoden sind im Sinne der Erfindung solche Methoden zu verstehen, bei denen ein isoliertes CGTase-Gen oder ein Fragment eines CGTase-Gens in an sich bekannter Art und Weise so modifiziert wird, daB ein Gen entsteht, welches für ein CGTase-Enzym kodiert, bei dem zumindest der Aminosäurerest, der homolog zu Tyr 229 in der CGTase aus Bacillus circulans ist, durch einen anderen Aminosäurerest, bevorzugt einen Tryptophanrest oder einen Serinrest, besonders bevorzugt einen Tryptophanrest, ersetzt wurde.

Aus dem Stand der Technik bekannte Verfahren zur "in vitro"-Mutagenese sind z. B. spezifische (BioTechniques (1992)13 (3), S. 342-346) oder unspezifische (Technique (1989) 1 (1), S. 11-15) Mutageneseverfahren mit Hilfe der "PCR"-Technik. Ebenso sind Verfahren bekannt, bei denen die Mutation gerichtet mit Hilfe eines synthetisierten Oligonukleotids in das Zielgen eingebracht wird. Dies kann sowohl mittels sogenannter "Einzelstrangverfahren" (Ausubel F.M et al. (1987) Current Protocols in Molecular Biology, Green Publishing Associates) oder auch mittels "Doppelstrangverfahren" (Promega 1992-1993 Catalogue, 150) oder mittels anderer Verfahren wie sie beispielsweise in Ann. Rev. Genet. (1985) 19, S. 423-462 beschrieben werden geschehen.

Die Verwendung zur Gewinnung von γ-CD aus Stärke ist das Hauptanwendungsgebiet der erfindungsgemäßen CGTase mit erhöhter γ-CD-Bildungsaktivität. Die erfindungsgemäßen CGTasen lassen sich dazu mittels gängiger Herstellungsverfahren nutzen. Gängige Herstellungsverfahren zur Produktion von γ-CD, In denen sich die erfindungsgemäßen CGTasen an Stelle der dort genannten CGTasen einsetzen lassen, sind zum Beispiel beschrieben bei:
- Journal of Fermentation and Bioengineering (1990) 70 (3), S. 190-192: Die Herstellung von γ-CD unter Verwendung der β-,γ-CD bildenden CGTase aus Bacillus sp. AL-6 in Gegenwart von Äthanol, welcher eine verstärkte γ-CD-Produktion bewirkt.
- JP 87 25,976: Die Herstellung von γ-CD unter Verwendung γ-CGTase aus Bacillus sp. 313.
- EP 291,067: Herstellung von γ-CD unter Verwendung der CGTase aus Bacillus macerans. Die Produktspezifität γ-CD wird durch die Zugabe eines Komplexbildners, z.B. Cyclohexadec-8-en-1-on, erreicht.
- DE 40 09 822: Produktion von γ-CD mit der γ-CGTase aus Bacillus sp. 290-3.

γ-CD besitzt sowohl im Vergleich zu α-CD als auch im Vergleich zu β-CD spezifische Vorteile, die es für eine Reihe von Anwendungen als einzig mögliches oder am besten geeignetes CD ausweisen.

Im Vergleich zu α-CD, das aus sechs Glukoseeinheiten aufgebaut ist, besitzt das aus acht Glukoseeinheiten bestehende γ-CD eine größere hydrophobe Kavität, die auch eine Komplexierung solcher Gastmoleküle ermöglicht, die aus sterischen Gründen von α-CD nicht komplexiert werden können.

Im Vergleich zu β-CD (Löslichkeit in Wasser bei Raumtemperatur: ca. 18,5 g/l) besitzt γ-CD eine wesentlich höhere Löslichkeit (bei Raumtemperatur: ca. 232,0 g/l) und ist damit für Komplexierungsreaktionen aus wäßrigen Lösungen besser geeignet als β-CD. Ein weiterer Vorteil von γ-CD gegenüber β-CD und modifizierten β-CD-Derivaten ist die geringe Toxizität von γ-CD. Sowohl bei oraler als auch bei intravenöser Applikation sind α-CD- und β-CD-Derivate im Tiermodell toxischer als γ-CD.

Die folgenden Beispiele dienen der weiteren Erläuterung der Erfindung.

### Beispiel 1: Mutagenese der γ-CGTase aus Bacillus sp. 290-3 (DSM 5850)

Der Austausch des Aminosäurerestes Tyrosin an der Position 211 in der γ-CGTase aus Bacillus sp. 290-3 (hinterlegt bei der Deutschen Sammlung für Mikroorganismen in Braunschweig unter der Nummer DSM 5850) gegen einen beliebigen anderen Aminosäurerest, insbesonders jedoch gegen einen Tryptophan- oder Serinrest, wird erreicht, indem das für die Tyrosin 211 kodierende Basentriplet des CGTase-Strukturgens in einer dem Fachmann an sich bekannten Art und Weise durch ein anderes Basentriplet, kodierend für einen beliebigen Aminosäurerest, vorzugsweise jedoch einenTryptophan rest, ersetzt wird.

Zur Mutagenese wurde das γ-CGTase-Gen aus Bacillus sp. 290-3 zunächst in den kommerziell erhältlichen E. coli-Vektor pUC19 (Boehringer, Mannheim) kloniert. Dazu wurdewie bei Ausubel F.M., Current protocols in molecular biology, vol. 1; Greene Publishing Associates & Wiley - Interscience, N. Y. beschrieben chromosomale DNS aus Bacillus sp. 290-3 (Proceedings of the 4th International Symposium on Cyclodextrins (1988) 87 - 92) isoliert und partiell mit der Restriktionsendonuklease Sau3AI (Boehringer, Mannheim) gespalten. Fragmente in einem Größenbereich zwischen zwei und fünf kb wurden isoliert und zusammen mit einer mit der Restriktionsendonuklease BamHI (Boehringer, Mannheim) linearisierten pUC19-DNS und T4 DNS-Ligase bei 16°C für 12 Stunden inkubiert. Der Ligationsansatz wurde zur Transformation von E. coli K 12-Zellen , die mit bekannten Verfahren (Maniatis, Molecular Cloning, A Laboratory Manual; Cold Spring Harbor Laboratory (1982), N.Y.) kompetent für die Aufnahme von DNS gemacht wurden, verwendet. Aus solchen E. coli-Zellen, die nach der Transformation auf Stärke enthaltenden Indikatorplatten Stärkeabbauhöfe bildeten, wurde das rekombinante Plasmid, welches das Gen für die γ-CGTase aus Bacillus sp. 290-3 trägt, isoliert (Maniatis, Molecular Cloning, A Laboratory Manual; Cold Spring Harbor Laboratory (1982), N.Y., S. 86-92).

Die Mutagenese dieses Gens wurde mit dem von der Firma Amersham (Braunschweig) kommerziell vertriebenen 'Oligonucleotide- directed in vitro mutagenesis system, version 2.1', basierend auf einem von Eckstein entwikkelten Verfahren (Nucl. Acids. Res. (1986) 14, S. 9679-9698 und Nucl. Acids. Res. (1988)16, S. 791-802) durchgeführt. Die Mutagenese wurde exakt nach dem Protokoll durchgeführt, welches dem genannten Mutagenese System der Fa. Amersham beiliegt. Das Verfahren wird im Folgenden summarisch wiedergegeben. Details sind dem Protokoll des genannten Mutagenesesystems zu entnehmen.

Unter Verwendung kommerziell erhältlicher Enzyme wie Restriktionsendonukleasen und T4 DNS-Ligase (Boehringer, Mannheim) wurde ein solcher Teil des in pUC19 klonierten Gens für die CGTase aus Bacillus sp. 290-3, der das für den Aminosäurerest Tyrosin an Position 211 dieser CGTase kodierende Basentriplet enthält, in den kommerziell erhältlichen Vektor M13 (New England Biolabs) kloniert. Ein Beispiel für ein solches Fragment ist ein 0,6 kb großes PstI/EcoRI-Fragment. Dieses Fragment wurde in den mit den Restriktionsendonukleasen Pstl und EcoRI gespaltenen M13-Vektor kloniert.

Aus solchen E. coli-Wirtszellen, die den rekombinanten M13-Vektor aufgenommen hatten, wurde nach der von Amersham mit dem oben genannten Mutagenese-System gelieferten Versuchsprotokoll einzelsträngige, rekombinante M13-DNS (Vorlagen-DNS) isoliert.

Zur eigentlichen Mutagenese wurden chemisch definierte Mutageneseollgonukleotlde mit jeweils erwünschter Sequenz synthetisiert. Solche Oligonukleotide sind beispielsweise bei der Fa. MWG (Ebersberg) käuflich erhältlich. Die Sequenz des Mutageneseoligonukleotids wurde so gewählt, daß die Abfolge der Basen in dem Mutageneseoligonukleotid invers komplementär zu dem Teil der Nukleotidsequenz der Vorlagen-DNS ist, der das in der Vorlagen-DNS enthaltene Basentriplet, kodierend für den Tyrosinrest an Position 211 γ-CGTase aus Bacillus sp. 290-3 jeweils 15 Basen stromauf und stromab umgibt. An Stelle des für Tyrosin kodierenden Basentriplets enthielt das Mutageneseoligonukleotid jedoch solche Nukleotide, die nach erfolgter Mutagenese zur Produktion von γ-CGTase-Derivaten führen, bei denen an Position 211 statt eines Tyrosinrestes ein anderer Aminosäurerest lokalisiert ist.

In Tab. 2 sind die Sequenzen der zwei verwendeten Mutageneseoligonukleotiden dargestellt.

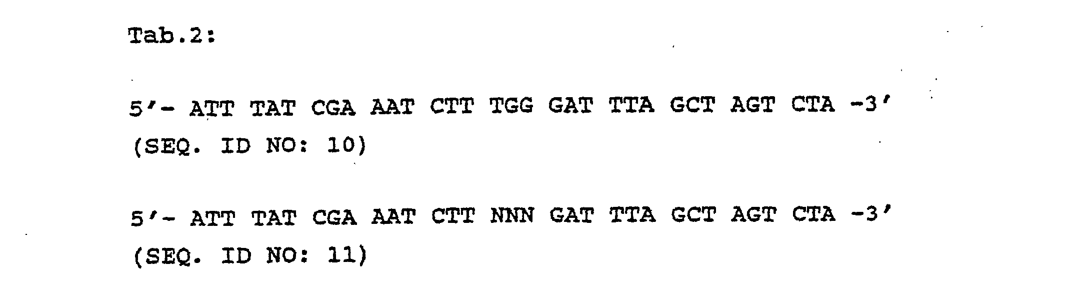

Der Einsatz des oberen in Tab.2 dargestellten Mutageneseoligonukleotids führte zu γ-CGTase-Derivaten, bei denen der Tyrosinrest 211 durch einen Tryptophanrest ersetzt war.

Bei Verwendung des unteren in Tab. 2 dargestellten Mutageneseoligonukleotids, einem sogenannten degenerierten-, oder 'mixed' - Oligonukleotid kann das fürTyrosin 211 kodierende Basentriplet durch jedes der 64 möglichen Basentriplets mit Ausnahme der für Tyr kodierenden Triplets ersetzt werden. Dieses Oligonukleotid ist daher geeignet zur Erzeugung von γ-CGTase-Derivaten, bei denen der Aminosäurerest Tyrosin an Position 211 durch jeweils eine der anderen natürlichen Aminosäuren ersetzt ist.

Die Mutageneseoligonukleotide wurden am 5'-Ende unter Verwendung von T4 Polynukleotid-Kinase und ATP (Amersham) phosphoryliert. Die phosphorylierten Mutageneseoligonukleotide wurden an die homologen Bereiche der Vorlagen-DNS gebunden. Dazu wurden 5 *µ*g einzelsträngiger Vorlagen-DNS mit ca. 4 pMol des phosphorylierten Mutageneseoligonukleotids für drei Minuten bei 70°C und dann für 30 Minuten bei 37°C inkubiert. Anschließend erfolgte die Synthese eines zur Vorlagen-DNS, mit Ausnahme der zu mutagenisierenden Position, komplementären DNS-Strangs, wobei das an die Vorlagen-DNS gebundene Mutageneseoligonukleotid als Startpunkt der Synthese und die Vorlagen-DNS als Vorlage für die Neusynthese des mutierten DNS-Strangs diente. Die Synthese selbst erfolgte nach Zugabe des Klenow-Fragments der DNA-Polymerase (Amersham), einer T4 DNS-Ligase und eines Nukleotidmixes, der die Nukleotide dATP, dGTP, dTTP und anstelle des dCTP das Thionukleotid dCTPαS (Amersham) enthält, über 15 Stunden bei 16°C.

Aus diesem Syntheseansatz wurden verbliebene Moleküle einzelsträngiger Vorlagen-DNS entfernt. Dazu wurde der Ansatz mit NaCl versetzt und über einen Nitrozellulosefilter (Amersham) filtriert, der spezifisch einzeisträngige DNS bindet. Die im Durchlauf verbliebene doppelsträngige Hybrid-DNS wurde durch eine EtOH-Fällung konzentriert und entsalzt. Anschließend wurde die Hybrid-DNS mit Ncil (Amersham), einer Restriktionsendonuklease, die die Nukleotidsequenz CC(G/C)GG erkennt, aber nur native DNS-Stränge, nicht jedoch solche, die das Nukleotid-Analogon dCTPαS enthalten, spaltet, in geeignetem Inkubationspuffer (Amersham) für 90 Minuten bei 37°C inkubiert. Durch diese Behandlung wurden nur in den nicht mutagenisierten Strang (Vorlagen-DNS) Brüche eingeführt.

Bei einer 30 minütigen Behandlung bei 37°C mit Exonuklease III (Amersham), einem Enzym, das DNS-Stränge von freien Enden her abbaut, wurde die Vorlagen-DNS dann entfernt. Nach einer thermischen Inaktivierung der Exonuklease III (15 Minuten bei 70°C) wurde der verbliebene, einzelsträngige und mutagenisierte DNS-Strang mit DNS-Polymerase I (Amersham), T4 DNS-Ligase und den Nukleotiden dATP, dTTP, dCTP und dGTP für 3 Stunden bei 16°C inkubiert. Dabei wurde die mutagenisierte Einzelstrang-DNS zum Doppelstrang ergänzt. Nach einer weiteren EtOH-Fällung zur Reinigung kann die mutagenisierte DNS in kompetente E. coli K12-Zellen transformiert werden.

Anschließend wurde das entsprechende, jedoch nicht mutagenisierte PstI/EcoRI-Fragmant aus dem auf pUC19 basierenden Expressionsplasmid für die γ-CGTase aus Bacillus sp. 290-3 herausgespalten und unter Verwendung von T4 DNS-Ligase durch das mutagenisierte Fragment ersetzt.

### Beispiel 2: Mutagenese der β-CGTase aus Bacillus circulans

Analog Beispiel 1 wurde das Kodon des β-CGTase-Gens aus Bacillus circulans, das für den Tyrosinrest an Position 229 der entsprechenden CGTase kodiert, durch ein Triplet ersetzt, das entweder für einen Tryptophanrest (Tab. 4 oben) oder einen Serinrest (Tab.4 unten) kodiert. Tab. 3 zeigt die Oligonukleotide, die für diese Mutagenesen verwendetwurden.

### Beispiel 3: Produktion der γ-CGTase aus Bacillus sp. 290-3 und ihrer erfindungsgemäßen Derivaten in E. coli

Zur Produktion der γ-CGTase aus Bacillus sp. 290-3 und ihrer gemäß Bsp. 1 hergestellten Derivate wurden die in Beispiel 1. beschriebenen Expressionsplasmide auf pUC19-Basis in einen E. coli-Sekretionsstamm transformiert. Als E. coli-Sekretionsstamm wurde E. coli WCM105 verwendet. Dieser Stamm wurde wie in EP 338410 beschrieben aus E. coli DS 410 hergestellt.

Zur Produktion γ-CGTase aus Bacillus sp. 290-3 oder deren Derivaten wurden daher Zellen von E. coli WCM105, die geeignete CGTase-Expressionsplasmide enthalten, in LB-Medium (Maniatis, Molecular Cloning, A Laboratory Manual; Cold Spring Harbor Laboratory (1982), N.Y.), das 10 g/l Laktose und 0,1 g/l Ampicillin enthält, für 72 Stunden bei 30°C in einem Wasserbadschüttler (Drehzahl 250 Upm) inkubiert. Dann wurden die Zellen durch Zentrifugation bei 5000 x g abgetrennt. Der zellfreie Kulturüberstand enthält die γ-CGTase oder deren Derivate.

### Beispiel 4: Produktion der β-CGTase aus Bacillus circulans und ihrer erfindungsgemäßen Derivate in E. coli

Die Produktion erfolgte analog Beispiel 3 unter Verwendung der in Bsp. 2 beschriebenen Expressionsplasmide.

### Beispiel 5: Konversion von Stärke zu Cyclodextrinen

Die Bestimmung der Aktivitäten von CGTasen erfolgte nach der in Eur. J. Biochem. (1990) 191, S. 177-185 beschriebenen Methode.

Jeweils 10 Einheiten pro Gramm Stärke einer zu testenden CGTase wurden mit einer 5 %-igen Lösung einer löslichen Stärke (Merck, Darmstadt) in einem Puffer bestehend aus 20 mM Tris/HCl pH 7,2 und 5 mM CaCl₂ für eine definierte Zeit bei 45°C inkubiert. Nach der Zeit wurde die Reaktion durch die Zugabe von 1,5 Volumenteilen Methanol beendet. Nicht umgesetzte Reststärke wurde durch eine einstündige Inkubation bei 4°C gefällt und durch Zentrifugation (10 min, 12000 x g) abgetrennt. Die entstandenen Produkte wurden über HPLC an einer Nukleosil 10-NH₂-Säule (Macherey & Nagel, Düren) bestimmt, wobei definierte Cyclodextrine oder lineare Maltooligosaccharide (Sigma, München) als Standard dienten.

### Beispiel 6: Konversion von Stärke unter Einsatz der nicht mutagenisierten γ-CGTase aus Bacillus sp. 290-3 sowie dem gemäß Bsp. 3 hergestellten Derivat.

Die Reaktionen wurden wie in Beispiel 5 beschrieben durchgeführt. Die Menge entstandener lineare Maltooligosaccharide (G1-G7) wurde aufaddiert. Die folgenden Resultate wurden erhalten:

**Tab. 4**

| Reaktionszeit in Minuten | Umsatz der Stärke zu Cyclodextrin und G1-G7 (%) | | | | | |
|---|---|---|---|---|---|---|
| | Nicht mutagenisierte CGTase | | | Mutagenisierte CGTase | | |
| | β-CD | γ-CD | G1-G7 | β-CD | γ-CD | G1-G7 |
| 5 | 7,0 | 8,6 | 0,0 | 0,0 | 7,8 | 0,0 |
| 10 | 11,0 | 13,0 | 0,0 | 0,0 | 12,8 | 0,0 |
| 15 | 12,6 | 14,4 | 0,0 | 0,0 | 16,2 | 0,0 |
| 30 | 21,4 | 18,2 | 1,2 | 2,0 | 22,8 | 2,2 |

### Beispiel 7: Konversion von Stärke unter Einsatz der nicht mutagenisierten β-CGTase aus Bacillus circulans sowie dem gemäß Bsp. 4 hergestellten Derivat, bei dem der Tyrosinrest an Position 229 durch einen Tryptophanrest ersetzt wurde

Die Reaktionen wurden wie in Beispiel 5 beschrieben durchgeführt. Die folgenden Resultate wurden erhalten:

**Tab. 5**

| Reaktionszeit in Minuten | Umsatz der Stärke zu Cyclodextrin (%) | | | | | |
|---|---|---|---|---|---|---|
| | Nicht mutagenisierte CGTase | | | Mutagenisierte CGTase | | |
| | α-CD | β-CD | γ-CD | α-CD | β-CD | γ-CD |
| 1 | 0,0 | 6,4 | 1,2 | 0,0 | 1,0 | 4,4 |
| 2 | 0,0 | 10,6 | 2,0 | 0,0 | 2,0 | 8,0 |
| 3 | 1,6 | 13,5 | 2,6 | 0,0 | 2,8 | 14,6 |
| 4 | 2,6 | 16,4 | 5,2 | 0,0 | 4,8 | 17,2 |
| 5 | 3,2 | 18,2 | 7,0 | 0,0 | 5,8 | 16,4 |
| 6 | 2,6 | 20,0 | 6,6 | 0,0 | 6,2 | 16,0 |
| 7 | 3,4 | 22,2 | 7,6 | 1,0 | 7,4 | 16,4 |
| 8 | 3,8 | 23,0 | 6,4 | 1,2 | 8,4 | 19,8 |
| 9 | 4,4 | 24,6 | 7,0 | 1,8 | 9,6 | 22,0 |
| 10 | 4,6 | 26,2 | 6,0 | 1,8 | 8,2 | 20,8 |

### Beispiel 8: Konversion von Stärke unter Einsatz der nicht mutagenisierten β-CGTase aus Bacillus circulans sowie dem gemäß Bsp. 4 hergestellten Derivat, bei dem der Tyrosinrest an Position 229 durch einen Tryptophanrest ersetzt wurde in Anwesenheit eines γ-CD-Komplexbildners

Die Konversion wurde wie in Bsp. 5 beschrieben mit den folgenden Modifikationen durchgeführt:
- Lösliche Stärke wurde durch Kartoffelstärke ersetzt
- 1,25 Gramm CHDC (Cyclohexadecenon) pro 10 Gramm Stärke wurden zugesetzt

Die folgenden Resultate wurden erhalten:

**Tab. 6**

| Reaktionszeit in Minuten | Umsatz der Stärke zu Cyclodextrin (%) | | | | | |
|---|---|---|---|---|---|---|
| | Nicht mutagenisierte CGTase | | | Mutagenisierte CGTase | | |
| | α-CD | β-CD | γ-CD | α-CD | β-CD | γ-CD |
| 1 | 2,3 | 18,0 | 5,4 | 1,4 | 10,5 | 13,7 |
| 2 | 3,2 | 20,0 | 7,2 | 2,2 | 13,8 | 22,2 |
| 3 | 4,4 | 21,8 | 9,5 | 3,4 | 15,8 | 23,7 |
| 4 | n.d. | n.d. | n.d. | 3,5 | 11,2 | 25,5 |
| 5 | 5,4 | 20,9 | 12,8 | 3,5 | 11,5 | 29,0 |
| 6 | 5,8 | 21,1 | 14,6 | n.d. | n.d, | n.d. |
| 7 | 6,4 | 20,6 | 17,0 | 4,7 | 11,0 | 32,0 |

### Beispiel 9: Konversion von Stärke unter Einsatz der nicht mutagenisierten β-CGTase aus Bacillus circulans sowie des erfindungsgemäßen Derivats aus Bsp. 4, bei dem der Tyrosinrest an Position 229 durch einen Serinrest ersetzt wurde

Die Reaktionen wurden wie in Beispiel 5 beschrieben durchgeführt. Bei einer 20 minütigen Inkubation wurden die folgenden Resultate erhalten:

**Tab. 7:**

| Umsatz der Stärke zu Cyclodextrin (%) | | | | | | |
|---|---|---|---|---|---|---|
| Reaktionszeit in Minuten | Nicht mutagenisierte CGTase | | | Mutagenisierte CGTase | | |
| | α-CD | β-CD | γ-CD | α-CD | β-CD | γ-CD |
| 20 | 4,3 | 20,1 | 6,6 | 1 | 13 | 13 |

## Patentansprüche

1. Mutierte Cyclodextringlycosyltransferase (CGTase)aus Bacillus sp. 290-3 oder Bacillus circulans, welche bei der Umsetzung von Stärke oder stärkeähnlichen Substraten zu Cyclodextrin (CD) in erhöhtem Ausmaß γ-CD produziert, **dadurch gekennzeichnet, daß** ihre Proteinsequenz im Bereich zwischen Aminosäureposition 180 und Aminosäureposition 240 die Aminosäuresequenz Asn Leu Xaa Asp (SEQ. ID NO: 1) enthält, wobei Position 1 der Proteinsequenz der Beginn des Signalpeptids der CGTase ist und Xaa Trp oder Ser bedeutet.

2. CGTase nach Anspruch 1, **dadurch gekennzeichnet, daß** Xaa trp bedeutet.

3. Verfahren zur Herstellung von CGTase, **dadurch gekennzeichnet, daß** die DNA Sequenz eines für eine β-oder γ-CGTase kodierenden Gens aus Bacillus sp. 290-3 oder Bacillus circulans mittels an sich bekannter Mutagenesemethoden derart mutiert wird, daß **dadurch** das im Bereich zwischen Aminosäurepostition 180 und 240 liegende Tyr im Sequenzmotiv Asn Leu Tyr Asp (SEQ. ID NO: 9) der eingesetzten β oder γ-CGTase in der mutierten CGTase gegen eine andere natürliche Aminosäure ausgetauscht ist.

4. Verfahren gemäß Anspruch 3, **dadurch gekennzeichnet, daß** das im Bereich zwischen Aminosäureposition 180 und 240 liegende Tyr im Sequenzmotiv Asn Leu Tyr Asp (SEQ. ID NO: 9) der eingesetzten β oder γ-CGTase in der mutierten CGTase gegen Trp oder Ser ausgetauscht ist

5. Verwendung von CGTasen nach Anspruch 1 oder 2 zur Produktion von γ-Cyclodextrin.

## Claims

1. Mutated cyclodextrin glycosyltransferase (CGTase) from Bacillus sp. 290-3 or Bacillus circulans which, when converting starch or starch-like substrates to cyclodextrin (CD), produces γ-CD to an increased extent, **characterized in that** its protein sequence, in the region between amino acid position 180 and amino acid position 240, contains the amino acid sequence Asn Leu Xaa Asp (SEQ. ID NO: 1), where position 1 of the protein sequence is the beginning of the signal peptide of the CGTase and Xaa denotes Trp or Ser.

2. CGTase according to Claim 1, **characterized in that** Xaa denotes Trp.

3. Process for preparing CGTases, **characterized in that** the DNA sequence of a gene encoding a β- or γ-CGTase from Bacillus sp. 290-3 or Bacillus circulans is mutated by means of mutagenesis methods which are known per se such that the Tyr situated in the region between amino acid positions 180 and 240 in the sequence motif Asn Leu Tyr Asp (SEQ. ID NO: 9) of the β- or γ-CGTase employed is thereby replaced in the mutated CGTase by another natural amino acid.

4. Process according to Claim 3, **characterized in that** the Tyr situated in the region between amino acid positions 180 and 240 in the sequence motif Asn Leu Tyr Asp (SEQ. ID NO: 9) of the β- or γ-CGTase employed is replaced in the mutated CGTase by Trp or Ser.

5. Use of CGTases according to Claim 1 or 2 for producing γ-cyclodextrin.

## Revendications

1. Cyclodextrineglycosyltransférase (CGTase) mutée dérivée de Bacillus sp. 290-3 ou de Bacillus circulans, qui produit dans une mesure élevée de la γ-CD lors de la transformation d'amidons ou de substrats analogues à de l'amidon en cyclodextrine (CD), **caractérisée en ce que** sa séquence protéinique, dans la zone entre la position d'acide aminé 180 et la position d'acide aminé 240, contient la séquence d'acides aminés Asn Leu Xaa Asp (SEQ. ID NO : 1), la position 1 de la séquence protéinique étant le début du peptide signal de la CGTase et Xaa représentant Trp ou Ser.

2. CGTase selon la revendication 1, **caractérisée en ce que** Xaa signifie Trp.

3. Procédé pour la préparation de CGTases, **caractérisé en ce que** la séquence d'ADN d'un gène dérivé de Bacillus sp. 290-3 ou de Bacillus circulans et codant pour une β-CGTase ou une γ-CGTase est mutée au moyen de méthodes de mutagenèse connues en soi de telle manière que la Tyr se trouvant dans la zone entre la position d'acide aminé 180 et la position d'acide aminé 240 dans le motif séquentiel Asn Leu Tyr Asp (SEQ. ID NO : 9) de la β-CGTase ou de la γ-CGTase utilisée est remplacée dans la CGTase mutée par un autre acide aminé naturel.

4. Procédé selon la revendication 3, **caractérisé en ce que** la Tyr se trouvant dans la zone entre la position d'acide aminé 180 et la position d'acide aminé 240 dans le motif séquentiel Asn Leu Tyr Asp (SEQ. ID NO : 9) de la β-CGTase ou de la γ-CGTase utilisée est remplacée dans la CGTase mutée par du Trp ou de la Ser.

5. Utilisation de CGTases selon les revendications 1 ou 2 pour la production de γ-cyclodextrine.


### SEQUENZPROTOKOLL [0059]

### (1) ALGEMEINE INFORMATION:

(i) ANMELDER:
   (A) NAME: Consortium fuer elektrochemische Industrie, GmbH
   (B) STRASSE: Zielstattstr. 20
   (C) ORT: Munich
   (E) LAND: Germany
   (F) POSTLEITZAHL: 81379
   (G) TELEPHON: 089/748440
   (H) TELEFAX: 089/74844350
   (I) TELEX: 5215553 cons d
(ii) ANMELDETITEL: Cyclodextringlykosyltransferasen zur Produktion von gamma-Cyclodextrin
(iii) ANZAHL DER SEQUENZEN: 14
(iv) COMPUTER-LESBARE FORM:
   (A) DATENTRÄGER: Floppy disk
   (B) COMPUTER: IBM PC compatible
   (C) BETRIEBSSYSTEM: PC-DOS/MS-DOS
   (D) SOFTWARE: PatentIn Release #1.0, Version #1.25 (EPA)

### (2) INFORMATION ZU SEQ ID NO: 1:

(i) SEQUENZ CHARAKTERISTIKA:
   (A) LÄNGE: 4 Aminosäuren
   (B) ART: Aminosäure
   (D) TOPOLOGIE: linear
(ii) ART DES MOLEKÜLS: Peptid
(v) ART DES FRAGMENTS: inneres
(xi) SEQUENZBESCHREIBUNG: SEQ ID NO: 1:
   Asn Leu Xaa Asp
   1

### (2) INFORMATION ZU SEQ ID NO: 4:

(i) SEQUENZ CHARAKTERISTIKA:
   (A) LÄNGE: 9 Aminosäuren
   (B) ART: Aminosäure
   (D) TOPOLOGIE: linear
(ii) ART DES MOLEKÜLS: Peptid
(v) ART DES FRAGMENTS: inneres
(vi) URSPRÜNLICHE HERKUNFT:
(A) ORGANISMUS: Bacillus circulans
(xi) SEQUENZBESCHREIBUNG: SEQ ID NO: 4:
   Tyr Lys Asn Leu Tyr Asp Leu Ala Asp
   1 5

### (2) INFORMATION ZU SEQ ID NO: 8:

(i) SEQUENZ CHARAKTERISTIKA:
   (A) LÄNGE: 9 Aminosäuren
   (B) ART: Aminosäure
   (D) TOPOLOGIE: linear
(ii) ART DES MOLEKÜLS: Peptid
(v) ART DES FRAGMENTS: inneres
(vi) URSPRÜNLICHE HERKUNFT:
   (A) ORGANISMUS: Bacillus sp. 290-3
(xi) SEQUENZBESCHREIBUNG: SEQ ID NO: 8:
   Tyr Arg Asn Leu Tyr Asp Leu Ala Ser
   1 5

### (2) INFORMATION ZU SEQ ID NO: 9:

(i) SEQUENZ CHARAKTERISTIKA:
   (A) LÄNGE: 4 Aminosäuren
   (B) ART: Aminosäure
   (D) TOPOLOGIE: linear
(ii) ART DES MOLEKÜLS: Peptid
(v) ART DES FRAGMENTS: inneres
(xi) SEQUENZBESCHREIBUNG: SEQ ID NO: 9:
   Asn Leu Tyr Asp
   1

### (2) INFORMATION ZU SEQ ID NO: 10:

(i) SEQUENZ CHARAKTERISTIKA:
   (A) LÄNGE: 33 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(xi) SEQUENZBESCHREIBUNG: SEQ ID NO: 10:
   ATTTATCGAA ATCTTTGGGA TTTAGCTAGT CTA
   33

### (2) INFORMATION ZU SEQ ID NO: 11:

(i) SEQUENZ CHARAKTERISTIKA:
   (A) LÄNGE: 30 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(xi) SEQUENZBESCHREIBUNG: SEQ ID NO: 11:

### (2) INFORMATION ZU SEQ ID NO: 13:

(i) SEQUENZ CHARAKTERISTIKA:
   (A) LÄNGE: 33 Basenpaare
   (B) ART: Nukleinsäure
   (C) STRANGFORM: Einzel
   (D) TOPOLOGIE: linear
(ii) ART DES MOLEKÜLS: DNS (genomisch)
(xi) SEQUENZBESCHREIBUNG: SEQ ID NO: 13:
   ATCTACAAAA ACCTGTGGGA CCTGGCCGAC TTC
   33

### (2) INFORMATION ZU SEQ ID NO: 14:

(i) SEQUENZ CHARAKTERISTIKA:
   (A) LÄNGE: 33 Basenpaare
   (B) ART: Nukleinsäure
   (C) STRANGFORM: Einzel
   (D) TOPOLOGIE: linear
(ii) ART DES MOLEKÜLS: DNS (genomisch)
(xi) SEQUENZBESCHREIBUNG: SEQ ID NO: 14:
   ATCTACAAAA ACCTGTCTGA CCTGGCCGAC TTC
   33